(19) Europäisches Patentamt
European Patent Office
Office européen des brevets

(11) Veröffentlichungsnummer: **0 400 429 A2**

# (12) EUROPÄISCHE PATENTANMELDUNG

(21) Anmeldenummer: **90109524.0**

(22) Anmeldetag: **19.05.90**

(51) Int. Cl.5: **C07D 498/04, //(C07D498/04, 265:00,265:00)**

(30) Priorität: **31.05.89 DE 3917602**

(43) Veröffentlichungstag der Anmeldung: **05.12.90 Patentblatt 90/49**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Patsch, Manfred, Dr.**
**Fritz-Wendel-Strasse 4**
**D-6706 Wachenheim(DE)**
Erfinder: **Marschner, Claus, Dr.**
**Franz-Boegler-Weg 3**
**D-6720 Speyer(DE)**

## (54) Verfahren zur Herstellung von Triphendioxazinen.

(57) Verfahren zur Herstellung von Triphendioxazinen durch Cyclisierung von Benzochinonderivaten in Schwefelsäure oder Oleum, wobei man die Cyclisierung in Gegenwart von Perborat, Percarbonat oder deren Mischungen als Oxidationsmittel durchführt.

EP 0 400 429 A2

Xerox Copy Centre

## Verfahren zur Herstellung von Triphendioxazinen

Die vorliegende Erfindung betrifft ein neues Verfahren zur Herstellung von Triphendioxazinen durch Cyclisierung von Benzochinonderivaten in Schwefelsäure oder Oleum mit Perborat, Percarbonat oder deren Mischungen als Oxidationsmittel.

Es ist bekannt, anionische Triphendioxazine durch Cyclisierung von 2,5-Diarylaminobenzochinonen, die in der Regel durch Umsetzung eines halogensubstituierten 1,4-Benzochinons mit der zweifach molaren Menge eines Arylamins hergestellt werden, unter Verwendung von Kondensationsmitteln, wie Schwefelsäure oder Oleum, gegebenenfalls in Gegenwart geeigneter Oxidationsmittel, z.B. Mangandioxid (EP-A-199 056), Natrium-, Kalium- oder Ammoniumperoxodisulfat (GB-A-1 589 915), Chlor, Brom oder anorganische Bromverbindungen (EP-A-296 411), Iod oder anorganische Iodverbindungen (US-A-4 532 323) oder katalytische Mengen organische Iodverbindungen (EP-A-311 969), herzustellen.

Aus der EP-A-292 906 ist weiter bekannt, die Cyclisierung in Oleum unter Zugabe von 35 gew.%iger wäßriger Wasserstoffperoxidlösung durchzuführen.

Bei diesen Verfahrensweisen wird jedoch häufig beobachtet, daß insbesondere bei solchen Diarylaminobenzochinonen, die im Arylrest des Arylamins Sulfonylgruppen enthalten, die Cyclisierungsreaktion schwierig durchzuführen ist und daß deshalb eine höhere Reaktionstemperatur sowie die Verwendung von höherprozentigem Oleum erforderlich ist. Aus diesem Grund treten dabei oft unerwünschte Nebenreaktionen, wie intra- oder intermolekulare Dehydratisierungsreaktionen, Kondensationsreaktionen, Hydrolyse oder oxidativer Abbau, auf, und es kommt zur Bildung von uneinheitlichen Reaktionsprodukten.

Desweiteren weist die Cyclisierung mittels 35 gew.%iger wäßriger wasserstoffperoxidlösung technische Nachteile auf, die sich aus dem höheren Sicherheitsaufwand sowie der starken Wärmetönung des Prozesses, die sich aus Reaktionswärme und Lösungswärme zusammensetzt, ergeben.

Aufgabe der vorliegenden Erfindung war es, ein neues Verfahren zur Herstellung von Triphendioxazinen bereitzustellen, bei dem die obengenannten Schwierigkeiten vermieden werden.

Es wurde nun gefunden, daß die Herstellung von Triphendioxazinen der Formel I

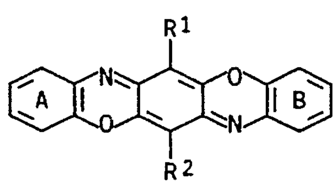 (I),

in der $R^1$ und $R^2$ gleich oder verschieden sind und unabhängig voneinander jeweils Wasserstoff, $C_1$-$C_4$-Alkyl, $C_1$-$C_4$-Alkoxy, Halogen, gegebenenfalls substituiertes Phenyl oder gegebenenfalls substituiertes Phenoxy bedeuten und die Ringe A und B gleich oder verschieden und unabhängig voneinander jeweils gegebenenfalls substituiert und/oder gegebenenfalls durch carbocyclische oder heterocyclische Ringe anelliert sind, durch Cyclisierung von Benzochinonen der Formel II

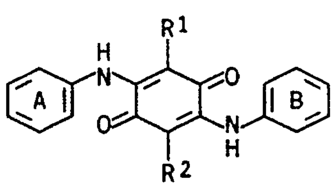 (II),

in der $R^1$, $R^2$ sowie die Ringe A und B jeweils die obengenannte Bedeutung besitzen, in Schwefelsäure oder Oleum als Reaktionsmedium und in Gegenwart eines Oxidationsmittels vorteilhaft gelingt, wenn man als Oxidationsmittel ein Perborat, ein Percarbonat oder deren Mischungen verwendet.

Alle in den obengenannten Formeln I und II auftretenden Alkylreste können sowohl geradkettig als auch verzweigt sein.

Wenn in den obengenannten Formeln I und II substituierte Phenylreste auftreten, können als Substituenten z.B. $C_1$-$C_4$-Alkyl, $C_1$-$C_4$-Alkoxy, Amino, Hydroxysulfonyl oder Halogen in Betracht kommen.

Wenn die Ringe A und/oder B substituiert sind, können als Substituenten (im folgenden als $R^3$

bezeichnet) z.B. gegebenenfalls substituiertes $C_1$-$C_4$-Alkyl, $C_5$-$C_7$-Cycloalkyl, Phenyl, Benzyl, $C_1$-$C_4$-Alkoxy, $C_5$-$C_7$-Cycloalkoxy, gegebenenfalls substituiertes Phenoxy, Phenylthio, Amino, gegebenenfalls substituiertes $C_1$-$C_4$-Alkylamino, $C_5$-$C_7$-Cycloalkylamino, gegebenenfalls substituiertes Phenylamino, Benzylamino, $C_1$-$C_4$-Alkanoylamino, Halogen, Carboxy, Hydroxysulfonyl, Sulfamoyl oder ein Rest der Formel

$-SO_2-R^4$ , 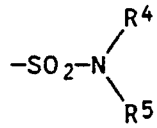 , 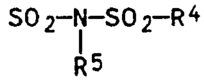 oder 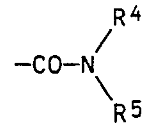

in Betracht kommen, wobei $R^4$ $C_1$-$C_6$-Alkyl, das gegebenenfalls durch Hydroxy, Halogen oder Sulfato substituiert ist, $C_2$-$C_4$-Alkenyl oder Phenyl und $R^5$ Wasserstoff, $C_1$-$C_6$-Alkyl, das gegebenenfalls durch Hydroxy, Halogen oder Sulfato substituiert ist, oder Phenyl bedeuten.

Wenn bei $R^3$ substituierte Phenylreste auftreten, können zusätzlich zu den obengenannten Substituenten, z.B. Hydroxy, $C_1$-$C_4$-Mono- oder Dialkylamino, Carboxy, $C_1$-$C_4$-Alkanoylamino, Sulfato oder 2-Sulfatoethylsulfonyl in Betracht kommen.

Die unter $R^3$ genannten Alkylreste können beispielsweise durch Amino, $C_1$-$C_4$-Mono- oder Dialkylamino, Hydroxy, $C_1$-$C_4$-Alkoxy, Hydroxysulfonyl, Carboxy, $C_1$-$C_4$-Alkanoylamino, Sulfato oder 2-Sulfatoethylsulfonyl substituiert sein.

Diejenigen Substituenten $R^3$, die über Sauerstoff oder Stickstoff an die Ringe A und/oder B gebunden sind, befinden sich dabei vorzugsweise in para-Stellung zum Stickstoffatom.

Reste $R^1$, $R^2$ und $R^3$ sind beispielsweise Methyl, Ethyl, Propyl, Isopropyl, Butyl, Isobutyl, sec-Butyl, Methoxy, Ethoxy, Propoxy, Isopropoxy, Butoxy, Isobutoxy, sec-Butoxy, Fluor, Chlor oder Brom.

Reste $R^1$ und $R^2$ sind weiterhin z.B. 4-Methylphenyl, 2,4-Dimethylphenyl, 2-Methoxyphenyl, 4-Ethoxyphenyl, 4-Isopropoxyphenyl, 4-Chlorphenyl, 4-Bromphenyl, 2,4-Dichlorphenyl, 2,6-Dichlorphenyl, 2-Methylphenoxy, 4-Methylphenoxy, 4-Methoxyphenoxy oder 4-Chlorphenoxy.

Reste $R^3$ sind weiterhin z.B. Cyclopentyl, Cyclohexyl, Cycloheptyl, Cyclopentyloxy, Cyclohexyloxy, Cycloheptyloxy, Phenoxy, 3-Aminophenoxy, 4-Aminophenoxy, Methylamino, Ethylamino, Propylamino, Isopropylamino, Butylamino, 2-Aminoethylamino, 3-Aminopropylamino, 3-Amino-2-methylpropylamino, 2-Hydroxyethylamino, 2-Hydroxysulfonylethylamino 2-Sulfatoethylamino, Cyclopentylamino, Cyclohexylamino, Cycloheptylamino, Phenylamino, 3-Aminophenylamino, 4-Aminophenylamino, Formylamino, Acetylamino, Propionylamino, Butyrylamino oder Isobutyrylamino.

Wenn die Ringe A und/oder B durch carbocyclische oder heterocyclische Ringe anelliert sind, kommen dafür beispielsweise der Benzoring, das Indanringsystem oder das Indolringsystem als Ringe in Betracht, wobei diese Ringsysteme gegebenenfalls durch Amino, $C_1$-$C_4$-Mono- oder Dialkylamino, $C_1$-$C_4$-Alkoxy, Hydroxysulfonyl, Carboxy, $C_1$-$C_4$-Alkanoylamino oder 2-Sulfatoethylsulfonyl weiter substituiert sind.

Für das erfindungsgemäße Verfahren geeignete Oxidationsmittel sind Perborate, Percarbonate oder deren Mischungen. Geeignete Perborate oder Percarbonate sind beispielsweise Metallperborate oder Metallpercarbonate. Vorzugsweise verwendet man Erdalkali- oder Alkalimetallperborate oder -percarbonate, z.B. Lithium-, Natrium-, Kalium-, Magnesium- oder Calciumperborat oder -percarbonat. Die Verwendung von Alkaliperborat- oder -percarbonat ist besonders bevorzugt, wobei insbesondere Natriumperborat oder-percarbonat hervorzuheben ist.

Bei diesen Oxidationsmitteln handelt es sich um an sich bekannte Verbindungen (vgl. W. Büchner, R. Schliebs, G. Winter "Industrielle Anorganische Chemie", Seite 27 und 28, Verlag Chemie, Weinheim, 1984).

Das Oxidationsmittel wird dabei in einer Menge von 2 bis 3 Mol, vorzugsweise 2 bis 2,2 Mol, jeweils bezogen auf 1 Mol Benzochinon II, verwendet.

Das erfindungsgemäße Verfahren wird bei einer Temperatur von 0 bis 50°C, vorzugsweise 5 bis 20°C durchgeführt.

Als Reaktionsmedium dient Schwefelsäure oder Oleum. Vorzugsweise arbeitet man in 90 bis 99 gew.%iger Schwefelsäure oder in 2 bis 10 gew.%igem Oleum.

Je Gewichtsteil zu cyclisierendes Benzochinon II werden im allgemeinen 5 bis 20 Gewichtsteile, vorzugsweise 7 bis 12 Gewichtsteile Schwefelsäure oder Oleum eingesetzt.

Es ist auch möglich, das neue Verfahren in Gegenwart eines Katalysators, z.B.Pentaacetylglucose, Tetraacetylethylendiamin oder Phthalsäureanhydrid, durchzuführen.

Das erfindungsgemäße Verfahren wird zweckmäßig so durchgeführt, daß man zunächst Schwefelsäure oder Oleum vorlegt, dann, gegebenenfalls unter Kühlen, das Benzochinonderivat der Formel II, Perborat, Percarbonat oder deren Mischungen zugibt und danach das Reaktionsgemisch über einen Zeitraum von

etwa 0,5 bis 5 Stunden bei der obengenannten Temperatur rührt. Anschließend gibt man das Reaktionsgemisch vorsichtig auf Eis. Das entstandene Triphendioxazin der Formel I wird abgesaugt, mit Wasser gewaschen und getrocknet.

Der Vorteil des erfindungsgemäßen Verfahrens ist darin zu sehen, daß die Reaktionstemperatur sehr niedrig ist, d.h. es laufen keine nachteiligen Nebenreaktionen ab und man gelangt in guter Ausbeute zu Produkten von hoher Reinheit und Qualität. Bei den im erfindungsgemäßen Verfahren zur Anwendung kommenden Oxidationsmittel handelt es sich außerdem um leicht zugängliche Produkte, die stabil und leicht handzuhaben sind.

Die Triphendioxazine der Formel I sind wertvolle Zwischenprodukte für die Synthese von Reaktivfarbstoffen und Papierfarbstoffen.

Die folgenden Beispiele sollen die Erfindung näher erläutern, die dort genannten Prozente sind Gewichtsprozente.

Beispiel 1

19,05g der Verbindung

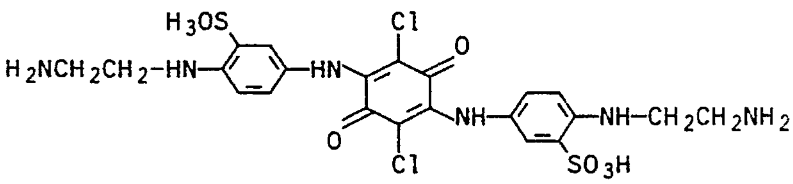

wurden bei 0 bis 10 °C in 150 g 96 %ige Schwefelsäure eingetragen. Nach Zugabe von 15,6 g Natriumperborat-trihydrat ($NaBO_2 \cdot H_2O_2 \cdot 3H_2O$) wurde 2 Stunden unter Kühlung bei 10 bis 15 °C nachgerührt. Nach Beendigung der Reaktion (Dünnschichtchromatographie) wurde das Reaktionsgemisch auf 1000 g Eis gefällt und mit Natronlauge auf einen pH-Wert von 6 gestellt. Nach Absaugen und Trocknen erhielt man 18,5 g eines blauen Pulvers, das überwiegend die Verbindungen der Formel

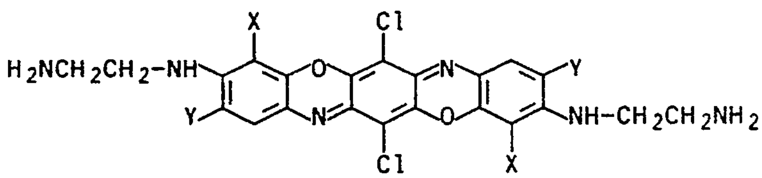

($\lambda_{max}$= 598 nm)

enthält, in der jeweils einer der Reste x und Y für Hydroxysulfonyl und der andere für Wasserstoff steht.

Beispiel 2

Es wurde analog Beispiel 1 gearbeitet. Jedoch verwendete man anstelle von Perborat 21 g Natriumpercarbonat ($2Na_2CO_3 \cdot 3H_2O_2$). Die Ausbeute betrug 17,5 g.

Beispiel 3

Man arbeitete analog Beispiel 1 jedoch verwendete man anstelle von Schwefelsäure 150 ml 2 %iges Oleum. Die Reaktionszeit betrug 0,5 Stunden. Aubeute: 18,5 g.

Beispiel 4

Man arbeitete analog Beispiel 3 jedoch verwendete man anstelle von Perborat 21 g Natriumpercarbonat ($2Na_2CO_3 \cdot 3H_2O_2$). Ausbeute: 18,0 g.

In analoger Weise werden die in der folgenden Tabelle aufgeführten Triphendioxazine der Formel

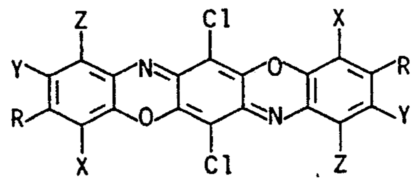

erhalten.

Tabelle

| Verb. Nr. | R | X, Y, Z | $\lambda_{max}$ [nm] |
|---|---|---|---|
| 5 | $NH-CH_2-CH(CH_3)-NH_2$ | $SO_3H$, H, H | 598 |
| 6 | $NH-(CH_2)_3-NH_2$ | $SO_3H$, H, H | 605 |
| 7 | 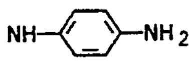 | $SO_3H$, H, H | 640 |
| 8 | $NH-(CH_2)_2-NH_2$ | $SO_2-(CH_2)_2-OSO_3H$, H, H | 615 |
| 9 | $NH-(CH_2)_2-SO_3H$ | $SO_2-(CH_2)_2-OSO_3H$, H, H | 610 |
| 10 | 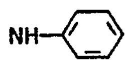 | $SO_2-(CH_2)_2-SO_3H$, H, H | 625 |
| 11 | $O-(CH_2)_2-NH_2$ | $SO_3H$, H, H | 515 |
| 12 | 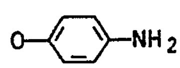 | $SO_3H$, H, H | 545 |

Tabelle (Fortsetzung)

| Verb. Nr. | R | X, Y, Z | $\lambda_{max}$ [nm] |
|---|---|---|---|
| 13 | $OCH_2-CH(OSO_3H)-CH_2SO_2C_2H_4OSO_3H$ | $SO_3H$, H, H | 508 |
| 14 | $NH_2$ | $SO_3H$, H, H | 590 |
| 15 | $NH_2$ | $SO_3H$, H, $SO_3H$ | 585 |
| 16 | $NH_2$ | $SO_3H$, H, $OCH_3$ | 600 |
| 17 | $NH-(CH_2)_2-NH_2$ | $CO-NH-C_2H_4OSO_3H$, H, H | 588 |
| 18 | $NH-(CH_2)_2-NH_2$ | $SO_2-NH-C_2H_4OSO_3H$, H, H | 605 |

**Ansprüche**

1. Verfahren zur Herstellung von Triphendioxazinen der Formel I

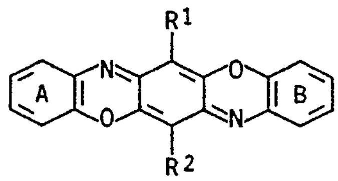

(I),

in der $R^1$ und $R^2$ gleich oder verschieden sind und unabhängig voneinander jeweils Wasserstoff, $C_1$-$C_4$-Alkyl, $C_1$-$C_4$-Alkoxy, Halogen, gegebenenfalls substituiertes Phenyl oder gegebenenfalls substituiertes Phenoxy bedeuten und die Ringe A und B gleich oder verschieden und unabhängig voneinander jeweils gegebenenfalls substituiert und/oder gegebenenfalls durch carbocyclische oder heterocyclische Ringe anelliert sind, durch Cyclisierung von Benzochinonen der Formel II

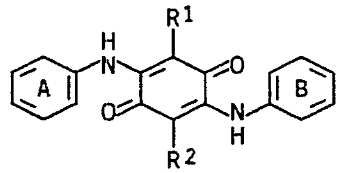

(II),

in der $R^1$, $R^2$ sowie die Ringe A und B jeweils die obengenannte Bedeutung besitzen, in Schwefelsäure oder Oleum als Reaktionsmedium und in Gegenwart eines Oxidationsmittels, dadurch gekennzeichnet, daß man als Oxidationsmittel ein Perborat, ein Percarbonat oder deren Mischungen verwendet.

2. Verfahren nach Anspruch 1, dadurch gekennzeichnet, daß man die Cyclisierung in Gegenwart von 2 bis 3 Mol, bezogen auf 1 Mol Benzochinon der Formel II, eines Oxidationsmittels durchführt.

3. Verfahren nach Anspruch 1, dadurch gekennzeichnet, daß man als Oxidationsmittel Alkaliperborat, Alkalipercarbonat oder deren Mischung verwendet.

4. Verfahren nach Anspruch 1, dadurch gekennzeichnet, daß man die Cyclisierung bei einer Temperatur von 0 bis 50 °C durchführt.

5. Verfahren nach Anspruch 1, dadurch gekennzeichnet, daß man die Cyclisierung in 90 bis 99 gew.%iger Schwefelsäure durchführt.

6. Verfahren nach Anspruch 1, dadurch gekennzeichnet, daß man die Cyclisierung in 2 bis 10 gew.%igem Oleum durchführt.